# EUROPEAN PATENT APPLICATION

(11) **EP 3 838 167 A1**
(43) Date of publication of application: **23.06.2021**
(21) Application number: 20214526.4
(22) Date of filing: 16.12.2020
(51) Int. Cl.: A61B 17/068

(54) **SURGICAL INSTRUMENT KITS FOR SEQUENCING USER OPERATIONS**

(30) Priority: 17.12.2019 US 201962949007 P; 08.10.2020 US 202017066261
(71) Applicant: Covidien LP, Mansfield, MA 02048 (US)
(72) Inventor: FISCHVOGT, Gregory W., Reno, NV 89521 (US)
(74) Representative: Maschio, Antonio

(57) **Abstract**

A kit for a powered surgical instrument causes the performance of a homing initiation procedure prior to the connection of a connectable component to the powered surgical instrument. The kit includes a powered surgical instrument, and end effector for coupling to the powered surgical instrument, and a blister pack housing at least the powered surgical instrument.

## Description

### CROSS-REFERENCE TO RELATED APPLICATION

The present application claims the benefit of and priority to U.S. Provisional Patent Application Ser. No. 62/949,007, filed on December 17, 2019, the entire content of which is incorporated herein by reference.

### FIELD

This disclosure relates to kits for powered surgical instruments, and in particular, powered surgical instrument kits that cause a powered surgical instrument to perform a homing initiating procedure prior to use of the powered surgical instrument.

### BACKGROUND

During laparoscopic or endoscopic surgical procedures, access to a surgical site is typically achieved through a small incision or through a narrow cannula inserted through a small entrance wound in a patient. Because of limited area to access the surgical site, many endoscopic surgical devices include mechanisms for articulating or rotating the tool assembly or the end effector of the device.

In surgical instruments that are used to apply tacks or anchors having helical threads, for example, an additional challenge exists when attempting to rotate the end effector, as the tacks are also configured to rotate through the end effector, through a surgical mesh, and into tissue, for instance. Some tack-applying surgical instruments include the ability for its end effector to articulate and rotate, while also limiting the overall amount of rotation to prevent the premature ejection of tacks and to prevent timing issues when attempting to eject tacks.

Some surgical instruments include a reusable handle that is usable with an attachable component, such as a tack cartridge that houses tacks. In such multi-component configurations, proper alignment of the interconnecting components ensures proper operation of the surgical instrument.

### SUMMARY

The disclosure relates to a kit for a powered surgical instrument. The kit includes a powered surgical instrument, a blister pack housing the powered surgical instrument, a component packaging housing an end effector configured to attach to the powered surgical instrument, and a non-conductive pull-tab connected to the powered surgical instrument and the component packaging. The powered surgical instrument includes a motor and a power source configured to power the motor and is configured to perform a homing initiation procedure upon a powering on thereof. The non-conductive pull-tab includes a proximal end electrically separating the motor of the powered surgical instrument from the power source configured to power the motor and a distal end coupled to a portion of the component packaging. The homing initiation procedure is commenced upon removal of the non-conductive pull-tab from between the motor and the power source to power on the powered surgical instrument.

In an aspect, the proximal end of the non-conductive pull-tab extends through a slit defined in a housing of the powered surgical instrument. The non-conductive pull-tab may be removably coupled to the powered surgical instrument and permanently coupled to the component packaging. In an aspect, the proximal end of the non-conductive pull-tab is disposed between a battery and a circuit coupled to the motor such that the non-conductive pull-tab prevents the formation of a circuit. Additionally, or alternatively, the distal end of the non-conductive pull-tab is coupled to the component packaging such that the end effector housed in the component packaging is incapable of being removed from the component packaging without removing the proximal end of the non-conductive pull-tab from the powered surgical instrument.

In an aspect, the powered surgical instrument is a tack applier.

In an aspect, the component packaging is positioned in the kit at least a distance from a distal end of the powered surgical instrument and a length of the non-conductive pull-tab is less than the distance such that the end effector housed in the component packaging is prevented from being able to be connected to the distal end of the powered surgical instrument without removing the proximal end of the non-conductive pull-tab from the powered surgical instrument.

In another aspect, a kit includes a powered surgical instrument, a blister pack housing the powered surgical instrument, a cover covering the blister pack and removable therefrom, and a non-conductive pull-tab connected to the powered surgical instrument and the cover. The powered surgical instrument includes a motor and a power source configured to power the motor and is configured to perform a homing initiation procedure upon a powering on thereof. The non-conductive pull-tab includes a proximal end electrically separating the motor of the powered surgical instrument from the power source configured to power the motor and a distal end coupled to a portion of the cover. The homing initiation procedure is commenced upon removal of the non-conductive pull-tab from between the motor and the power source to power on the powered surgical instrument.

In an aspect, the proximal end of the non-conductive pull-tab extends through a slit defined in a housing of the powered surgical instrument. The non-conductive pull-tab may be removably coupled to the powered surgical instrument and permanently coupled to the cover. In an aspect, the proximal end of the non-conductive pull-tab is disposed between a battery and a circuit coupled to the motor such that the non-conductive pull-tab prevents the formation of a circuit. Additionally, or alternatively, the distal end of the non-conductive pull-tab is coupled to the cover such that the powered surgical instrument is incapable of being separated from the cover without removing the proximal end of the non-conductive pull-tab from the powered surgical instrument.

In an aspect, the powered surgical instrument is a tack applier.

In another aspect, a kit includes a powered surgical instrument, a blister pack housing the powered surgical instrument, and a non-conductive pull-tab connected to the powered surgical instrument and the blister pack. The powered surgical instrument includes a motor and a power source configured to power the motor and is configured to perform a homing initiation procedure upon a powering on thereof. The non-conductive pull-tab includes a proximal end electrically separating the motor of the powered surgical instrument from the power source configured to power the motor and a distal end coupled to a portion of the blister pack. The homing initiation procedure is commenced upon removal of the non-conductive pull-tab from between the motor and the power source to power on the powered surgical instrument.

In an aspect, the proximal end of the non-conductive pull-tab extends through a slit defined in a housing of the powered surgical instrument. The non-conductive pull-tab may be removably coupled to the powered surgical instrument and permanently coupled to the blister pack. In an aspect, the proximal end of the non-conductive pull-tab is disposed between a battery and a circuit coupled to the motor such that the non-conductive pull-tab prevents the formation of a circuit. Additionally, or alternatively, the distal end of the non-conductive pull-tab is coupled to the blister pack such that the powered surgical instrument is incapable of being separated from the blister pack without removing the proximal end of the non-conductive pull-tab from the powered surgical instrument.

In an aspect, the powered surgical instrument is a tack applier. Additionally, or alternatively, the kit may include an end effector including a plurality of tacks configured to couple to the powered surgical instrument.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

Various aspects of the disclosure are described hereinbelow with reference to the drawings, which are incorporated and constitute a part of this specification, wherein:
FIG. 1 is a perspective view of a handle assembly of a powered surgical tack applier in accordance with an aspect of the disclosure;
FIG. 2 is a partial perspective view of an elongate member of the powered surgical tack applier;
FIG. 3 is a partial perspective view of a loading unit of the surgical tack applier of FIG. 1, illustrating a coil separated from an inner tube;
FIG. 4 is a longitudinal, cross-sectional view of a distal end of the powered surgical tack applier, illustrating implanting of a surgical tack into underlying tissue through a surgical mesh;
FIG. 5 is a perspective view of a surgical mesh for use with the powered surgical tack applier of FIG. 1, illustrating anchoring the surgical mesh to underlying tissue with a plurality of surgical tacks;
FIG. 6 is a side view of the handle assembly of FIG. 1 with a half of a housing removed;
FIG. 7 is an exploded perspective view of the handle assembly of FIG. 1 with parts separated;
FIG. 8 is a partial side view of the handle assembly of FIG. 1;
FIG. 9 is a partial side view of the handle assembly of FIG. 1 with a portion of the housing removed;
FIG. 10 is a partial perspective view of the handle assembly of FIG. 1, illustrating an actuation assembly;
FIG. 11 is a perspective view of a handle assembly for use with a powered surgical tack applier in accordance with another aspect of the disclosure;
FIG. 12 is a perspective view of the handle assembly of FIG. 11 with a half of the housing removed;
FIG. 13 is a side view of the handle assembly of FIG. 11;
FIG. 14 is a perspective view of a powered surgical tack applier in accordance with an aspect of the disclosure;
FIG. 15 is a kit of this disclosure for the powered surgical instrument of FIGS. 1 and 14;
FIG. 16 is another kit of this disclosure for the powered surgical instrument of FIGS. 1 and 14; and
FIG. 17 is another kit of this disclosure for the powered surgical instrument of FIGS. 1 and 14.

### DETAILED DESCRIPTION

In electrically powered laparoscopic surgical devices, there often is a need to perform a homing routine before a user operates the device. Prior to use, there may also be an operation where a user needs to add a consumable component/subassembly to the device prior to use, for example, a reload loading unit that is attachable to the shaft of the instrument. In such instances, the proper sequencing of events can help ensure the proper operation of the instrument and the firing of the components from the reload. For instance, if a reload is attached prior to homing, the device may not deliver the correct output or may be entirely inoperable.

Although homing routines are typically performed on the surgical devices prior to their packaging and shipment, it is possible that components of the device may move during shipment or handling of the packaged device. In such instances, the device would benefit from an additional homing routine prior to use. Users may not be aware of whether movement of components occurred during shipment and handling of the packed device, and therefore may not perform a homing routine on the device before operation thereof.

The packaging solutions (also referred to as kits), described herein solve the above-noted problem. In particular, the present packaging solutions prevent the operation of the device until a homing routine is performed. In battery powered laparoscopic surgical devices, a strip of non-conductive plastic may be used to separate the battery contacts from the positive and/or negative terminals of the battery. This breaks the circuit and insures the device is stable throughout its shelf life. As described in greater detail below, sizing of the non-conductive strip and placement relative to the surgical device and other components of a kit address the above-identified problems in that the connectable component is incapable of being connected to the surgical device until the homing routine is performed. In an aspect, the length of the plastic strip is substantially shorter than the distance required to install the reload. This forces the user to pull the strip, powering the device and allowing the homing routine to auto start prior to being able to install the reload on the device.

Embodiments of the presently disclosed kits are described in detail with reference to the drawings, in which like reference numerals designate identical or corresponding elements in each of the several views. As used herein the term "distal" refers to that portion of the endoscopic surgical device that is farther from the user, while the term "proximal" refers to that portion of the endoscopic surgical device that is closer to the user.

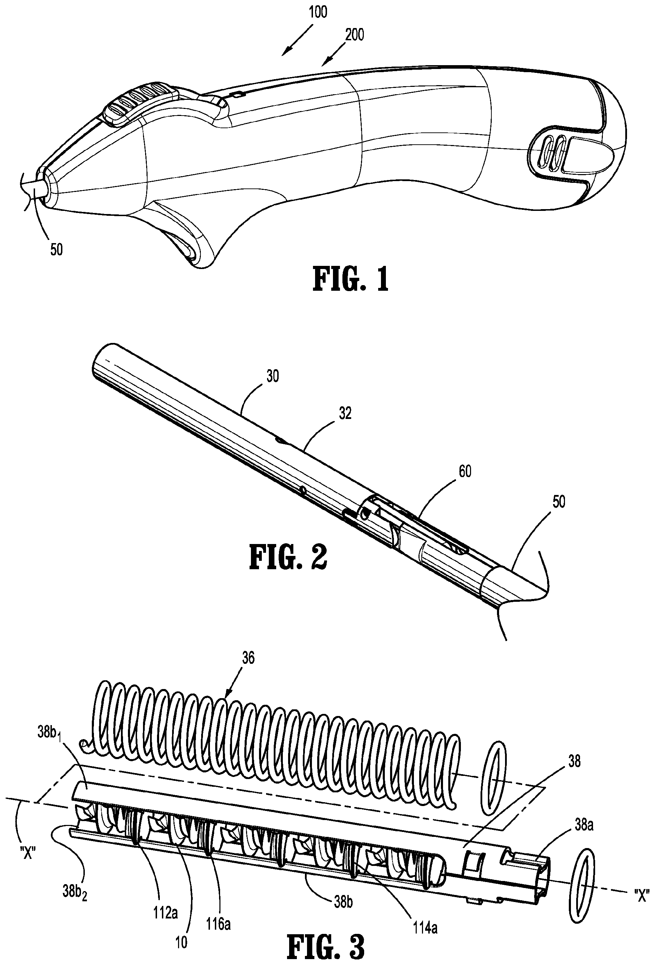

With reference to FIGS. 1-4, a handle assembly for use with a powered surgical instrument 100, for example a powered surgical tack applier for applying a surgical tack 10 suitable for insertion through a surgical mesh "M" and tissue "T" is shown generally as a handle assembly 200. The surgical tack applier generally includes the handle assembly 200, an elongate member 50 having an articulation portion 60, and a loading unit 30 selectably connectable to a distal end of the elongate member 50. The loading unit 30 is electro-mechanically coupled to the handle assembly 200 and supports a plurality of surgical tacks 10.

The loading unit 30 includes an outer tube 32 defining a lumen (not shown), a spiral or coil 36 fixedly disposed within the outer tube 32, and an inner tube 38 rotatably disposed within the coil 36. The inner tube 38 defines a lumen therethrough, and includes a first portion 38a and a splined second portion 38b. The second portion 38b of the inner tube 38 is slotted, defining a pair of tines 38bi and a pair of channels 38b₂. The second portion 38b of the inner tube 38 is configured to support the plurality of surgical tacks 10 within the inner tube 38. In particular, the surgical tacks 10 are loaded into the loading unit 30 such that the pair of opposing threaded sections 112a of the surgical tacks 10 extend through respective channels 38b₂ of the second portion 38b of the inner tube 38 and are slidably disposed within the groove of the coil 36, and the pair of tines 38b₁ of the second portion 38b of the inner tube 38 are disposed within the pair of slotted sections 116a of the surgical tack 10. In use, as the inner tube 38 is rotated about a longitudinal axis "X-X" thereof, relative to the coil 36, the pair of tines 38bi of the inner tube 38 transmits the rotation to the surgical tacks 10 and advances the surgical tacks 10 distally as the head threads 114a of the surgical tacks 10 engage with the coil 36.

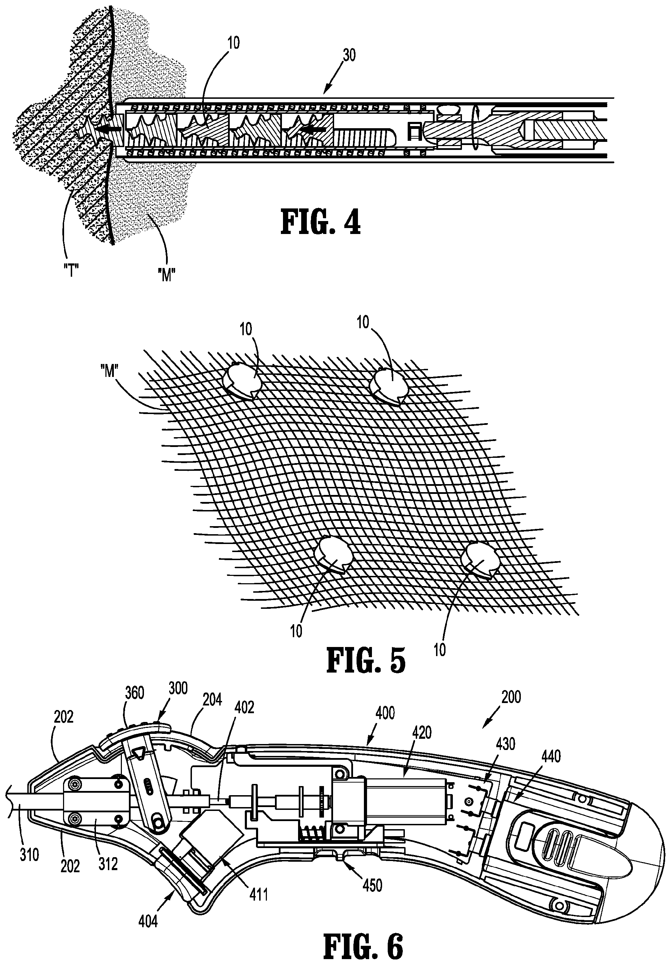

With particular respect to FIG. 2, the surgical tack applier includes an articulation portion 60 operatively coupled with an articulation lever assembly 300 (FIG. 6) supported in the handle assembly 200. The articulation portion 60 may include a drive assembly (not shown) having a slidable tube and an articulation arm pivotally coupled to the slidable tube. The articulation lever assembly 300 is coupled to the slidable tube so that when the articulation lever assembly 300 is actuated the slidable tube is displaced through the elongated member 50. Longitudinal translation of the slidable tube moves the articulation arm to enable the loading unit 30 to articulate relative to the longitudinal axis "X-X" (FIG. 3).

With reference now to FIG. 6, the handle assembly 200 includes a housing 202, an articulation lever assembly 300 configured to articulate the articulation portion 60 (FIG. 2) of the elongate member 50, an actuation assembly 400 configured to eject the surgical tack 10 out of the loading unit 30 of the elongate member 50, and a battery pack 440 removably attached to the housing 202. The housing 202 includes an ergonomic structure providing comfort, ease of use, and intuitiveness such that when the housing 202 is gripped by a clinician, e.g., a thumb, may be positioned to slide the articulation lever assembly 300 and, e.g., an index finger, may be positioned to trigger an actuation switch 404 of the actuation assembly 400. Actuation of the actuation assembly 400 ejects a surgical tack 10 (FIG. 4) out of the loading unit 30 through mesh "M" (FIG. 4) and into body tissue "T".

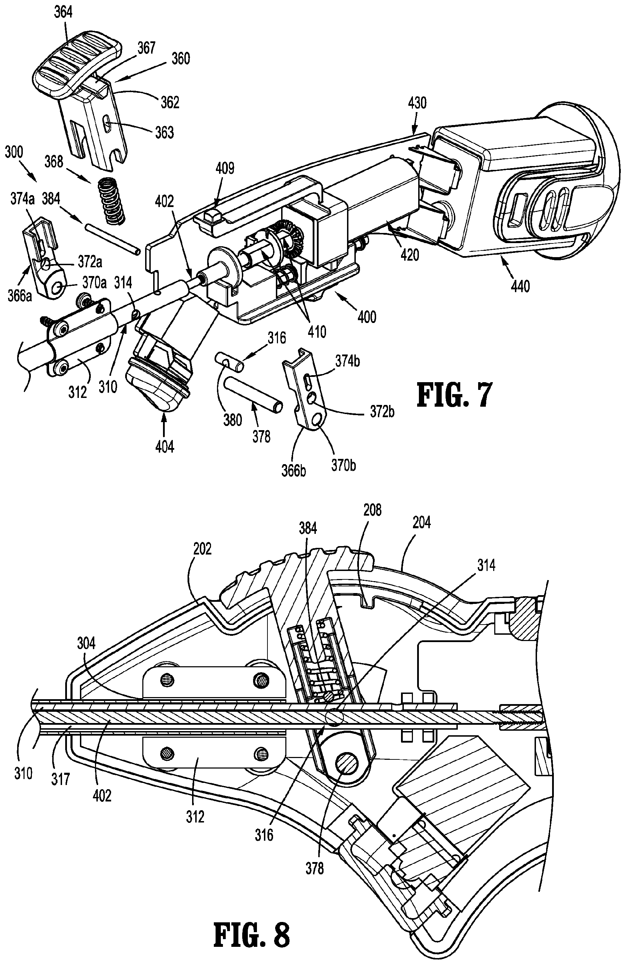

With reference to FIGS. 6 and 7, the articulation lever assembly 300 includes an articulation rod 310 and articulation lever 360 operatively coupled with the articulation rod 310. The articulation rod 310 is operatively coupled with the articulation portion 60 (FIG. 2) of the elongate member 50 of the surgical tack applier. The articulation rod 310 is slidably supported on the housing 202 of the handle assembly 200 by a mounting plate 312 defining a channel 304 (FIG. 8) configured to enable axial displacement of the articulation rod 310 therethrough, which, causes articulation of the articulation portion 60 (FIG. 2) based on the axial position of the articulation rod 310. In particular, the articulation rod 310 has an annular structure defining a channel 317 (FIG. 8) dimensioned to receive the actuation rod 402 of the actuation assembly 400 therein. The articulation rod 310 further defines a transverse bore 314 dimensioned to receive an articulation drive pin 316 coupled with the articulation lever 360.

With continued reference to FIGS. 6 and 7, the articulation lever 360 includes a housing portion 362 and an engaging portion 364 slidably engaging an engaging surface 204 of the housing 202. The engaging surface 204 has an arcuate profile enabling the engaging portion 364 to travel in, e.g., an arc. The housing portion 362 is disposed within the housing 202 and is dimensioned to receive articulation pivot arms 366a, 366b mated together to receive a biasing member 368 therebetween. Each articulation pivot arm 366a, 366b defines a first bore 370a, 370b, a second bore 372a, 372b, and a slot 374a, 374b. The first bores 370a, 370b are dimensioned to receive an articulation pivot pin 378 (FIG. 8) pivotably coupling the articulation pivot arms 366a, 366b to the housing 202. The second bores 372a, 372b are dimensioned to receive the articulation drive pin 316 extending through the transverse bore 314 of the articulation rod 310. Under such a configuration, when the articulation pivot arms 366a, 366b are pivoted about the articulation pivot pin 378, the articulation drive pin 316 causes axial displacement of the articulation rod 310. The articulation drive pin 316 defines a transverse bore 380 dimensioned to receive the actuation rod 402 of the actuation assembly 400 therethrough. The slots 374a, 374b of the articulation pivot arms 366a, 366b are dimensioned to cammingly receive a cam pin 384 biased away from the articulation pivot pin 378 by a biasing member 368 interposed between the articulation pivot arms 366a, 366b.

With reference now to FIGS. 7 and 8, the housing portion 362 of the articulation lever 360 is dimensioned to receive the mated articulation pivot arms 366a, 366b. The housing portion 362 defines a slot 363 dimensioned to cammingly receive the cam pin 384 which is cammingly slidable in the slots 374a, 374b of the articulation pivot arms 366a, 366b. In addition, the housing portion 362 includes a tooth 367 configured to engage a detent portion 208 of the housing 202 to inhibit movement of the articulation lever 360 relative to the housing 202, thereby locking an axial position of the articulation rod 310, which, in turn, locks the orientation of the articulation portion 60 (FIG. 2) of the surgical tack applier. Under such a configuration, the articulation lever 360 is biased away from the articulation pivot pin 378 such that the tooth 367 of the housing portion 362 engages the detent portion 208. When the engaging portion 364 of the articulation lever 360 is depressed towards the housing 202, the tooth 367 is moved away from the detent portion 208 enabling the clinician to slidably move the engaging portion 364 on the engaging surface 204 (FIG. 6) of the housing 202, thereby enabling articulation of the articulation portion 60 of the surgical tack applier to a desired orientation.

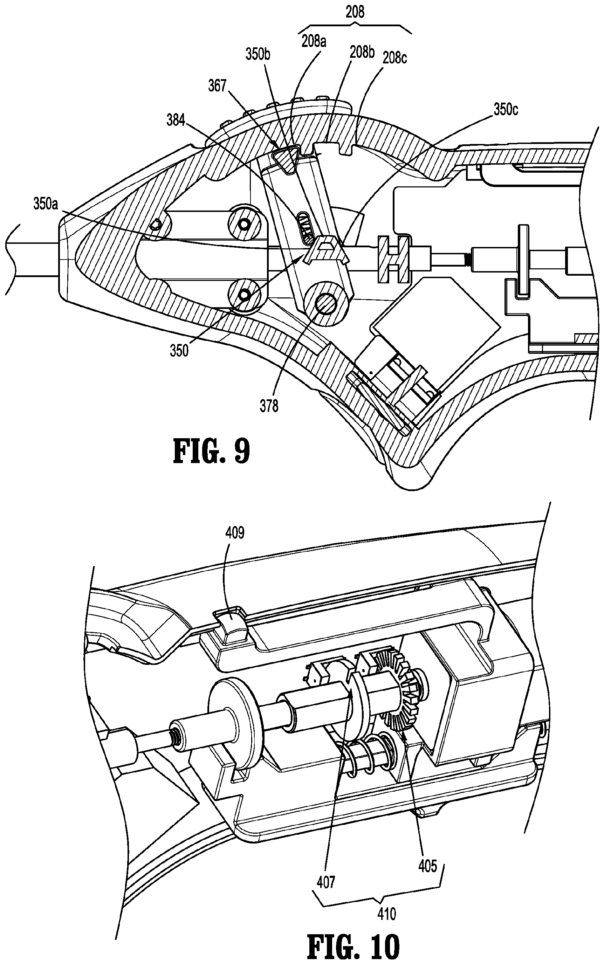

With reference now to FIG. 9 the articulation lever assembly 300 further includes a cam wedge 350 having first, second, and third portions 350a, 350b, 350c configured to cammingly engage the cam pin 384 which is cammingly slidable in the slots 374a, 374b of the articulation pivot arms 366a, 366b and the slot 363 of the articulation lever 360. The first, second, and third portions 350a, 350b, 350c correspond to the respective detent sections 208a, 208b, 208c of the detent portion 208. In this manner, articulation backlash is reduced as the cam pin 384 rides along the first, second, and third portions 350a, 350b, 350c of the cam wedge 350.

With reference back to FIGS. 6 and 7, the actuation assembly 400 includes an actuation rod 402 operatively coupled with the loading unit 30 (FIG. 2) of the surgical tack applier, a motor 420, an actuation switch 404 configured to actuate the motor 420 to eject the surgical tacks 10 (FIG. 4), a printed circuit board 430 including a microprocessor (not shown) to control the actuation assembly 400, and a battery pack 440 removably attached to the housing 202 and electrically connected to the motor 420 and the printed circuit board 430. A proximal end of the actuation rod 402 is operatively coupled with an output shaft of the motor 420 for concomitant rotation therewith such that when the actuation switch 404 is triggered by the clinician, the motor 420 is actuated to impart axial rotation to the actuation rod 402. A distal end of the actuation rod 402 is operatively coupled with the inner tube 38 (FIG. 3) of the loading unit 30 for concomitant rotation therewith.

With reference now to FIG. 10, the actuation assembly 400 may further include an encoder assembly 410 operatively connected to the actuation rod 402 and the processor of the printed circuit board 430. The encoder assembly 410 may include, e.g., an optical, motor encoder 405 configured to keep an accurate count of turns of the motor output shaft or the actuation rod 402 to ensure a proper number of turns are made to insert the surgical tack 10 through, e.g., the mesh "M", and into tissue "T" (FIG. 4). In addition, the encoder assembly 410 may further include, e.g., a single notched, encoder wheel 407 configured to ensure correct clocking of a distal end of the actuation rod 402 relative to the loading unit 30 (FIG. 2). The encoder assembly 410 may further include a light emitting diode ("LED") indicator 409 to indicate status of the ejection of each surgical tack 10. For example, a green light may indicate proper application of the surgical tack 10 through the mesh "M" and into tissue "T", and a red light may indicate, e.g., improper application of the surgical tack 10, due to an error signal from the optical motor encoder 405 or the single notched encoder wheel 407. Alternatively, the encoder assembly 410 may further include a piezoelectric element 411 (FIG. 6) for providing an audible tone for proper application of the surgical tack 10.

With brief reference to FIG. 6, the handle assembly 200 may further include a release lever 450 slidably attached to the housing 202. The release lever 450 is operatively coupled with the loading unit 30 (FIG. 2) such that when the release lever 450 is pulled, the loading unit 30 is detached from the elongate member 50 (FIG. 2) of the surgical tack applier.

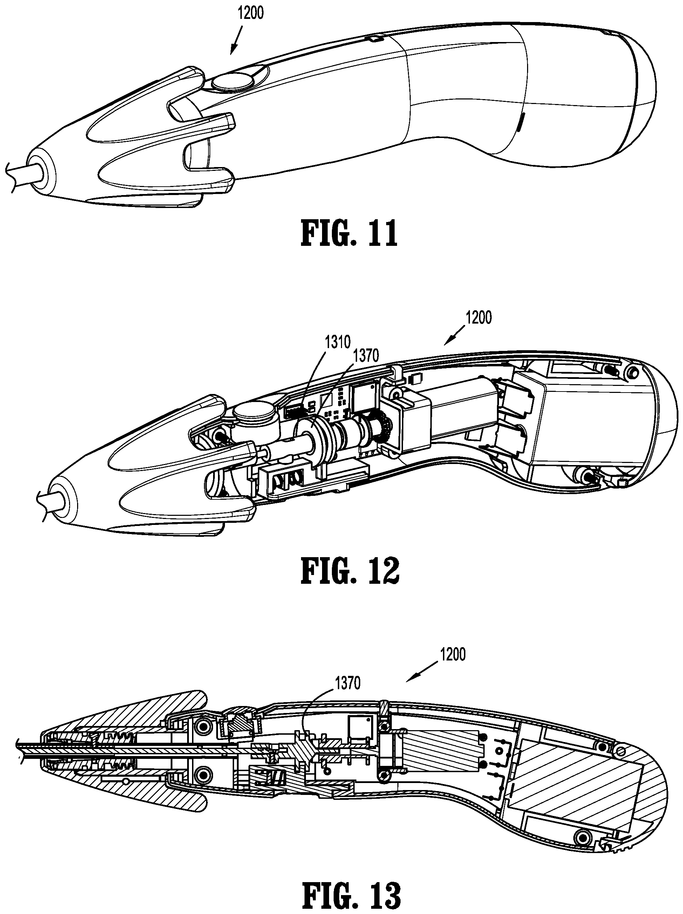

In use, the loading unit 30 is operatively mounted to a distal end of the elongate member 50. The loading unit 30 is introduced into a target surgical site while in the non-articulated condition. The clinician may remotely articulate loading unit 30 relative the longitudinal axis "X-X" to access the surgical site. Specifically, the clinician may slide the engaging portion 364 of the articulation lever 360 along the engaging surface 204 of the housing 202. As the articulation rod 310 is displaced axially, the loading unit 30 is moved to an articulated orientation relative to the central longitudinal axis "X-X". Furthermore, the clinician may position the surgical mesh "M" adjacent the surgical site. Once the surgical mesh "M" is properly positioned on the surgical site, the clinician may trigger the actuation switch 404 to eject a surgical tack 10 through the mesh "M" and into tissue "T". While the articulation rod 310 is configured for axial displacement, it is further contemplated that an actuation rod 1310 may be rotatably supported by a rotor 1370 such that the actuation rod 1310 outputs an axial rotation which may be utilized by the loading unit 30 to effect articulation thereof, as can be appreciated with reference to FIGS. 11-13. It is further contemplated that the actuation assembly 400 may further include a transmission assembly to selectively impart rotation of the output shaft of the motor 420 to the actuation rod 1310.

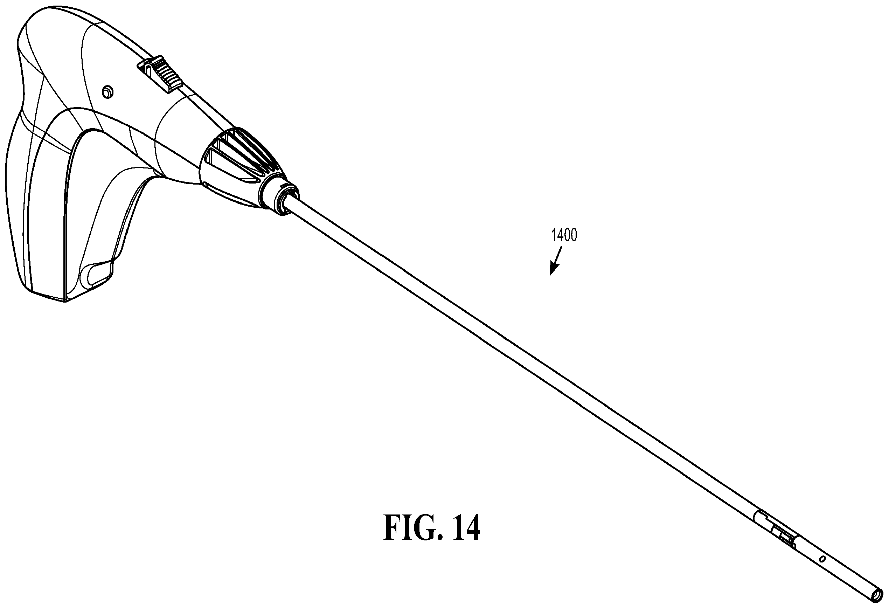

FIG. 14 illustrates another powered surgical instrument, for example a powered surgical tack applier, shown as powered surgical instrument 1400. Powered surgical instrument 1400 includes similar electronic components as powered surgical instrument 100, and includes similar detachable loading units, and will not be described in further detail for brevity.

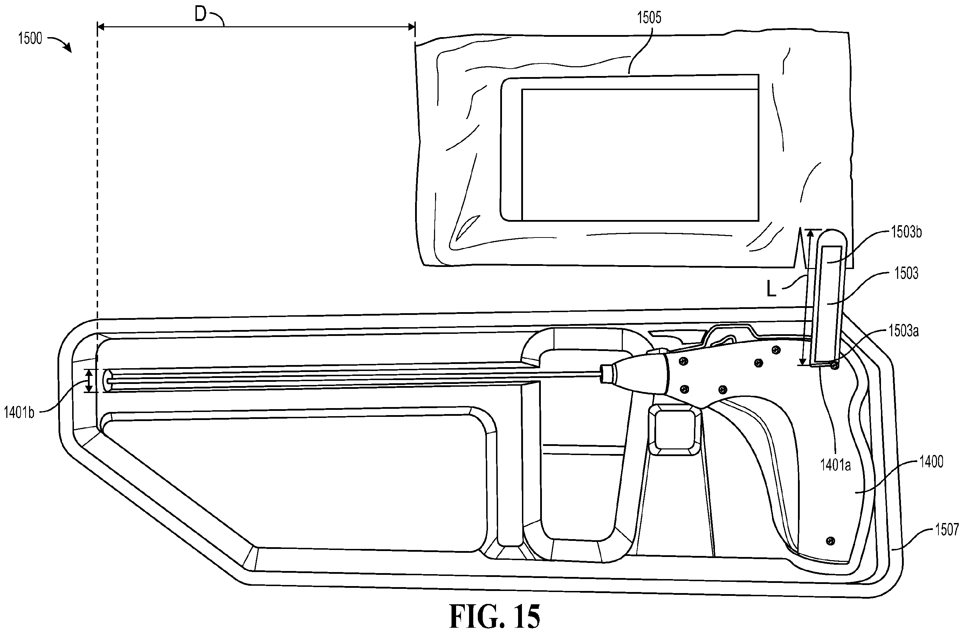

Referring now to FIG. 15, a kit 1500 of this disclosure includes a powered surgical instrument 1400 housed within a blister pack 1507 along with a component packaging 1505. The powered surgical instrument 1400 includes a motor (e.g., motor 420 illustrated in FIG. 7) and is configured to perform a homing initiation procedure upon powering on. The component packaging 1505 houses a component that is configured to attach to a distal end 1401b of the powered surgical instrument 1400, for example a loading unit 30 (FIG. 2). A non-conductive pull-tab 1503 is connected to the powered surgical instrument 1400 and the component packaging 1505. The non-conductive pull-tab 1503 includes a proximal end 1503a electrically separating the motor (not shown) of the powered surgical instrument 1400 from a power source (e.g., battery) configured to power the motor and a distal end 1503b coupled to a portion of the component packaging 1505. In an aspect, the non-conductive pull-tab 1503 electrically separates a power source (e.g., battery) from circuitry including a processor coupled to a motor (not shown) when in the initial, packaged, position. For example, the non-conductive pull-tab 1503 may be electrically isolating a power source (e.g., battery) or leads connected to a power source from control circuitry coupled to, and configured to control, a motor (not shown). In such a case, when the non-conductive pull-tab 1503 is removed from the powered surgical instrument 1400, the control circuit becomes energized by the power source (e.g., battery) and the processor of the control circuitry performs a homing initiation procedure on the motor to ensure proper position and orientation thereof.

The non-conductive pull-tab 1503 is removably coupled to the powered surgical instrument 1400 and permanently coupled to the component packaging 1505. Specifically, the proximal end 1503a of the non-conductive pull-tab 1503 extends through a slit 1401a defined in a housing of the powered surgical instrument 1400 and is disposed between a battery and a circuit coupled to the motor such that the non-conductive pull-tab 1503 prevents the formation of a circuit within the electronic components of the powered surgical instrument 1400. With this configuration, removal of the non-conductive pull-tab 1503 from the powered surgical instrument 1400 causes the powered surgical instrument 1400 to perform a homing initiation procedure to calibrate the position of the motor and gears connected to the motor.

The distal end 1503b of the non-conductive pull-tab 1503 is coupled to the component packaging 1505 such that the component (e.g., loading unit 30, FIG. 2) housed in the component packaging 1505 is incapable of being removed from the component packaging 1505, and incapable of being attached to the powered surgical instrument 1400, without removing the proximal end 1503a of the non-conductive pull-tab 1503 from the powered surgical instrument 1400.

With this configuration, a user is prevented from connecting the component housed in the component packaging 1505 unless the non-conductive pull-tab 1503 is removed from the powered surgical instrument 1400, thereby initiating the homing initiation procedure, and in particular, forces a sequence of operations to take place in a specific order that ensures that the attachable component (e.g., loading unit 30, FIG. 2) can be attached to the powered surgical instrument 1400 only after the homing initiation procedure is performed. In particular, the component packaging 1505 is positioned in the kit 1500 at least a distance "D" from a distal end 1401b of the powered surgical instrument 1400 and a length "L" of the non-conductive pull-tab 1503 is less than the distance "D" such that the component housed in the component packaging 1505 is prevented from being able to be connected to the distal end 1401b of the powered surgical instrument 1400 without removing the proximal end 1503a of the non-conductive pull-tab 1503 from the powered surgical instrument 1400.

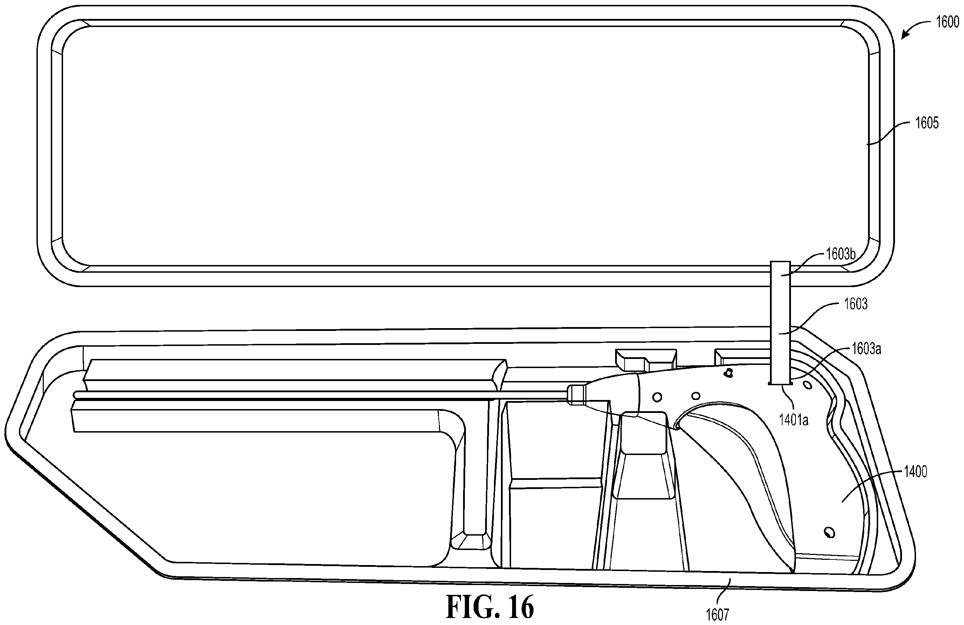

FIG. 16 illustrates another kit 1600 which includes a powered surgical instrument 1400 housed within a blister pack 1607 and enclosed by a cover 1605 covering the blister pack 1607. The cover 1605 is peelable from the blister pack 1607 to access the powered surgical instrument 1400 from the blister pack 1607. In kit 1600, a non-conductive pull-tab 1603 is connected to the powered surgical instrument 1400 and the cover 1605. The non-conductive pull-tab 1603 includes a proximal end 1603a electrically separating the motor of the powered surgical instrument 1400 with a power source configured to power the motor and a distal end 1603b coupled to a portion of the cover 1605.

The non-conductive pull-tab 1603 is removably coupled to the powered surgical instrument 1400 and permanently coupled to the cover 1605. The proximal end 1603a of the non-conductive pull-tab 1603 extends through a slit 1401a defined in a housing of the powered surgical instrument 1400. In particular, the proximal end 1603a of the non-conductive pull-tab 1603 is disposed between a battery and a circuit coupled to the motor such that the non-conductive pull-tab 1603 prevents the formation of a circuit. As described above with respect to non-conductive pull-tab 1503 (FIG. 15), in an aspect, the non-conductive pull-tab 1603 electrically separates a power source (e.g., battery) from circuitry including a processor coupled to a motor (not shown) when in the initial, packaged, position. For example, the non-conductive pull-tab 1603 may be electrically isolating a power source (e.g., battery) or leads connected to a power source from control circuitry coupled to, and configured to control, a motor (not shown). In such a case, when the non-conductive pull-tab 1603 is removed from the powered surgical instrument 1400, the control circuit becomes energized by the power source (e.g., battery) and the processor of the control circuitry performs a homing initiation procedure on the motor to ensure proper position and orientation thereof.

The distal end 1603b of the non-conductive pull-tab 1603 is coupled to the cover 1605 such that the powered surgical instrument 1400 is incapable of being separated from the cover 1605 without removing the proximal end 1603a of the non-conductive pull-tab 1603 from the powered surgical instrument 1400. Removal of the non-conductive pull-tab 1603 from the powered surgical instrument 1400 causes the powered surgical instrument 1400 to perform a homing initiation procedure to calibrate the position of the motor and the gears connected to the motor.

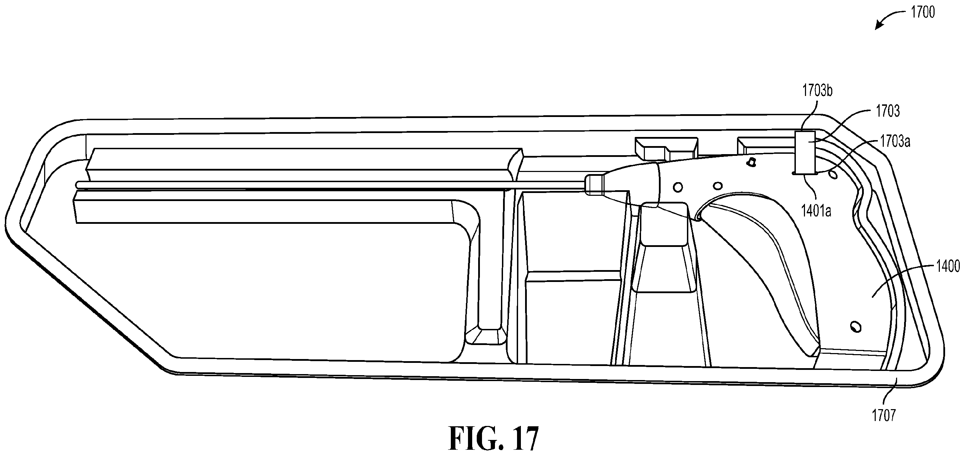

FIG. 17 illustrates another kit 1700 which includes a powered surgical instrument 1400 housed within a blister pack 1707. In kit 1700, a non-conductive pull-tab 1703 is connected to the powered surgical instrument 1400 and the blister pack 1707 or other sterilization pouches, for example a pouch or parts thereof that may be breathable or non-breathable. Thus, although the term "blister pack" is used herein, it is understood that blister pack 1707 may include a preformed rigid material, a preformed non-rigid material, other sterilization pouches whether preformed or not preformed, or combinations thereof. The non-conductive pull-tab 1703 includes a proximal end 1703a electrically separating the motor of the powered surgical instrument 1400 with a power source configured to power the motor and a distal end 1703b coupled to a portion of the blister pack 1707.

As described above with respect to non-conductive pull-tab 1503 (FIG. 15) and non-conductive pull-tab 1603 (FIG. 16), in an aspect, the non-conductive pull-tab 1703 electrically separates a power source (e.g., battery) from circuitry including a processor coupled to a motor (not shown) when in the initial, packaged, position. For example, the non-conductive pull-tab 1703 may be electrically isolating a power source (e.g., battery) or leads connected to a power source from control circuitry coupled to, and configured to control, a motor (not shown). In such a case, when the non-conductive pull-tab 1703 is removed from the powered surgical instrument 1400, the control circuit becomes energized by the power source (e.g., battery) and the processor of the control circuitry performs a homing initiation procedure on the motor to ensure proper position and orientation thereof.

The non-conductive pull-tab 1703 is removably coupled to the powered surgical instrument 1400 and permanently coupled to the blister pack 1707. The proximal end 1703a of the non-conductive pull-tab 1703 extends through a slit 1401a defined in a housing of the powered surgical instrument 1400 and is disposed between a battery and a circuit coupled to the motor such that the non-conductive pull-tab 1703 prevents the formation of a circuit. The distal end 1703b of the non-conductive pull-tab 1703 is coupled to the blister pack 1707 such that the powered surgical instrument 1400 is incapable of being separated from the blister pack 1707 without removing the proximal end 1703a of the non-conductive pull-tab 1703 from the powered surgical instrument 1400. Removal of the non-conductive pull-tab 1703 from the powered surgical instrument 1400 causes the powered surgical instrument 1400 to perform a homing initiation procedure to calibrate the position of the motor and the gears connected to the motor.

The packaging configurations, also described as kits, described above for the performance of a homing initiation procedure for the components of a powered surgical instrument prior to its use, and in particular, prior to the connection of a loading unit to the powered surgical instrument. Such a forced sequencing of user operations ensures the safe and proper operation of the instruments being used.

It will be understood that various modifications may be made to the embodiments disclosed herein. Therefore, the above description should not be construed as limiting, but merely as exemplifications of various embodiments. Those skilled in the art will envision other modifications within the scope and spirit of the claims appended thereto.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

The invention may be described by reference to the following numbered paragraphs:-
1. A kit for a powered surgical instrument, the kit comprising:
   a powered surgical instrument including a motor and a power source configured to power the motor, the powered surgical instrument configured to perform a homing initiation procedure upon a powering on thereof;
   a blister pack housing the powered surgical instrument;
   a component packaging housing an end effector configured to attach to the powered surgical instrument;
   a non-conductive pull-tab connected to the powered surgical instrument and the component packaging, the non-conductive pull-tab including a proximal end electrically separating the motor of the powered surgical instrument from the power source configured to power the motor and a distal end coupled to a portion of the component packaging, wherein the homing initiation procedure is commenced upon removal of the non-conductive pull-tab from between the motor and the power source to power on the powered surgical instrument.
2. The kit according to paragraph 1, wherein the proximal end of the non-conductive pull-tab extends through a slit defined in a housing of the powered surgical instrument.
3. The kit according to paragraph 1, wherein the non-conductive pull-tab is removably coupled to the powered surgical instrument and permanently coupled to the component packaging.
4. The kit according to paragraph 1, wherein the proximal end of the non-conductive pull-tab is disposed between a battery and a circuit coupled to the motor such that the non-conductive pull-tab prevents the formation of a circuit.
5. The kit according to paragraph 1, wherein the distal end of the non-conductive pull-tab is coupled to the component packaging such that the end effector housed in the component packaging is incapable of being removed from the component packaging without removing the proximal end of the non-conductive pull-tab from the powered surgical instrument.
6. The kit according to paragraph 1, wherein the powered surgical instrument is a tack applier.
7. The kit according to paragraph 1, wherein the component packaging is positioned in the kit at least a distance from a distal end of the powered surgical instrument and a length of the non-conductive pull-tab is less than the distance such that the end effector housed in the component packaging is prevented from being able to be connected to the distal end of the powered surgical instrument without removing the proximal end of the non-conductive pull-tab from the powered surgical instrument.
8. A kit for a powered surgical instrument, the kit comprising:
   a powered surgical instrument including a motor and a power source configured to power the motor, the powered surgical instrument configured to perform a homing initiation procedure upon a powering on thereof;
   a blister pack housing the powered surgical instrument;
   a cover covering the blister pack and removable therefrom;
   a non-conductive pull-tab connected to the powered surgical instrument and the cover, the non-conductive pull-tab including a proximal end electrically separating the motor of the powered surgical instrument from the power source configured to power the motor and a distal end coupled to a portion of the cover, wherein the homing initiation procedure is commenced upon removal of the non-conductive pull-tab from between the motor and the power source to power on the powered surgical instrument.
9. The kit according to paragraph 8, wherein the proximal end of the non-conductive pull-tab extends through a slit defined in a housing of the powered surgical instrument.
10. The kit according to paragraph 8, wherein the non-conductive pull-tab is removably coupled to the powered surgical instrument and permanently coupled to the cover.
11. The kit according to paragraph 8, wherein the proximal end of the non-conductive pull-tab is disposed between a battery and a circuit coupled to the motor such that the non-conductive pull-tab prevents the formation of a circuit.
12. The kit according to paragraph 8, wherein the distal end of the non-conductive pull-tab is coupled to the cover such that the powered surgical instrument is incapable of being separated from the cover without removing the proximal end of the non-conductive pull-tab from the powered surgical instrument.
13. The kit according to paragraph 8, wherein the powered surgical instrument is a tack applier.
14. A kit for a powered surgical instrument, the kit comprising:
   a powered surgical instrument including a motor and a power source configured to power the motor, the powered surgical instrument configured to perform a homing initiation procedure upon a powering on thereof;
   a blister pack housing the powered surgical instrument;
   a non-conductive pull-tab connected to the powered surgical instrument and the blister pack, the non-conductive pull-tab including a proximal end electrically separating the motor of the powered surgical instrument from the power source configured to power the motor and a distal end coupled to a portion of the blister pack, wherein the homing initiation procedure is commenced upon removal of the non-conductive pull-tab from between the motor and the power source to power on the powered surgical instrument.
15. The kit according to paragraph 14, wherein the proximal end of the non-conductive pull-tab extends through a slit defined in a housing of the powered surgical instrument.
16. The kit according to paragraph 14, wherein the non-conductive pull-tab is removably coupled to the powered surgical instrument and permanently coupled to the blister pack.
17. The kit according to paragraph 14, wherein the proximal end of the non-conductive pull-tab is disposed between a battery and a circuit coupled to the motor such that the non-conductive pull-tab prevents the formation of a circuit.
18. The kit according to paragraph 14, wherein the distal end of the non-conductive pull-tab is coupled to the blister pack such that the powered surgical instrument is incapable of being separated from the blister pack without removing the proximal end of the non-conductive pull-tab from the powered surgical instrument.
19. The kit according to paragraph 14, wherein the powered surgical instrument is a tack applier.
20. The kit according to paragraph 14, further comprising an end effector including a plurality of tacks, the end effector configured to couple to the powered surgical instrument.

## Claims

1. A kit for a powered surgical instrument, the kit comprising:
a powered surgical instrument including a motor and a power source configured to power the motor, the powered surgical instrument configured to perform a homing initiation procedure upon a powering on thereof;
a blister pack housing the powered surgical instrument;
a component packaging housing an end effector configured to attach to the powered surgical instrument;
a non-conductive pull-tab connected to the powered surgical instrument and the component packaging, the non-conductive pull-tab including a proximal end electrically separating the motor of the powered surgical instrument from the power source configured to power the motor and a distal end coupled to a portion of the component packaging, wherein the homing initiation procedure is commenced upon removal of the non-conductive pull-tab from between the motor and the power source to power on the powered surgical instrument.

2. The kit according to claim 1, wherein the proximal end of the non-conductive pull-tab extends through a slit defined in a housing of the powered surgical instrument.

3. The kit according to claim 1 or claim 2, wherein the non-conductive pull-tab is removably coupled to the powered surgical instrument and permanently coupled to the component packaging.

4. The kit according to any preceding claim, wherein the proximal end of the non-conductive pull-tab is disposed between a battery and a circuit coupled to the motor such that the non-conductive pull-tab prevents the formation of a circuit; and/or wherein the distal end of the non-conductive pull-tab is coupled to the component packaging such that the end effector housed in the component packaging is incapable of being removed from the component packaging without removing the proximal end of the non-conductive pull-tab from the powered surgical instrument.

5. The kit according to any preceding claim, wherein the powered surgical instrument is a tack applier.

6. The kit according to any preceding claim, wherein the component packaging is positioned in the kit at least a distance from a distal end of the powered surgical instrument and a length of the non-conductive pull-tab is less than the distance such that the end effector housed in the component packaging is prevented from being able to be connected to the distal end of the powered surgical instrument without removing the proximal end of the non-conductive pull-tab from the powered surgical instrument.

7. A kit for a powered surgical instrument, the kit comprising:
a powered surgical instrument including a motor and a power source configured to power the motor, the powered surgical instrument configured to perform a homing initiation procedure upon a powering on thereof;
a blister pack housing the powered surgical instrument;
a cover covering the blister pack and removable therefrom;
a non-conductive pull-tab connected to the powered surgical instrument and the cover, the non-conductive pull-tab including a proximal end electrically separating the motor of the powered surgical instrument from the power source configured to power the motor and a distal end coupled to a portion of the cover, wherein the homing initiation procedure is commenced upon removal of the non-conductive pull-tab from between the motor and the power source to power on the powered surgical instrument.

8. The kit according to claim 7, wherein the proximal end of the non-conductive pull-tab extends through a slit defined in a housing of the powered surgical instrument; and/or wherein the non-conductive pull-tab is removably coupled to the powered surgical instrument and permanently coupled to the cover.

9. The kit according to claim 7 or claim 8, wherein the proximal end of the non-conductive pull-tab is disposed between a battery and a circuit coupled to the motor such that the non-conductive pull-tab prevents the formation of a circuit; and/or wherein the distal end of the non-conductive pull-tab is coupled to the cover such that the powered surgical instrument is incapable of being separated from the cover without removing the proximal end of the non-conductive pull-tab from the powered surgical instrument.

10. The kit according to any of claims 7 to 9, wherein the powered surgical instrument is a tack applier.

11. A kit for a powered surgical instrument, the kit comprising:
a powered surgical instrument including a motor and a power source configured to power the motor, the powered surgical instrument configured to perform a homing initiation procedure upon a powering on thereof;
a blister pack housing the powered surgical instrument;
a non-conductive pull-tab connected to the powered surgical instrument and the blister pack, the non-conductive pull-tab including a proximal end electrically separating the motor of the powered surgical instrument from the power source configured to power the motor and a distal end coupled to a portion of the blister pack, wherein the homing initiation procedure is commenced upon removal of the non-conductive pull-tab from between the motor and the power source to power on the powered surgical instrument.

12. The kit according to claim 11, wherein the proximal end of the non-conductive pull-tab extends through a slit defined in a housing of the powered surgical instrument; and/or wherein the non-conductive pull-tab is removably coupled to the powered surgical instrument and permanently coupled to the blister pack.

13. The kit according to claim 11 or claim 12, wherein the proximal end of the non-conductive pull-tab is disposed between a battery and a circuit coupled to the motor such that the non-conductive pull-tab prevents the formation of a circuit.

14. The kit according to any of claims 11 to 13, wherein the distal end of the non-conductive pull-tab is coupled to the blister pack such that the powered surgical instrument is incapable of being separated from the blister pack without removing the proximal end of the non-conductive pull-tab from the powered surgical instrument.

15. The kit according to any of claims 11 to 14, wherein the powered surgical instrument is a tack applier; preferably further comprising an end effector including a plurality of tacks, the end effector configured to couple to the powered surgical instrument.
